Europäisches Patentamt

European Patent Office

Office européen des brevets

(11) Publication number: **0 224 453 B1**

(12) **EUROPEAN PATENT SPECIFICATION**

(45) Date of publication of patent specification: **09.09.92**  (51) Int. Cl.5: **C08L  89/06,** A61K 47/42, A61K 7/00, A61L 15/16

(21) Application number: **86830347.0**

(22) Date of filing: **17.11.86**

(54) Biologically active topical collagen support matrix: cosmetic and pharmaceutical uses and a process for its preparation.

(30) Priority: **27.11.85 IT 4882585**

(43) Date of publication of application:
**03.06.87 Bulletin  87/23**

(45) Publication of the grant of the patent:
**09.09.92 Bulletin  92/37**

(84) Designated Contracting States:
**AT BE CH DE ES FR GB GR LI LU NL SE**

(56) References cited:
**WO-A-85/04413**      **DE-A- 2 348 685**
**GB-A- 2 032 777**     **GB-A- 2 131 293**
**US-A- 3 991 184**      **US-A- 4 412 947**

(73) Proprietor: **Morganti, Pierfrancesco
49, Via Montoggio
I-00168 Roma RM(IT)**

(72) Inventor: **Morganti, Pierfrancesco
49, Via Montoggio
I-00168 Roma RM(IT)**

(74) Representative: **Bazzichelli, Alfredo
c/o Società Italiana Brevetti S.p.A. Piazza di
Pietra, 39
I-00186 Roma(IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention describes a support matrix for cosmetic or pharmaceutical use consisting of collagen which is partially naturally reticulated and insoluble and partially water soluble. When brought into contact with the skin, this support matrix has the property of transferring into the skin a considerable quantity of water from the insensible perspiration of the skin. This support matrix is therefore suitable for use in the treatment of dry skin and for facilitating the penetration of active substances into the skin.

Collagen is a well known constituent of white fibrous tissue in animals. It is found in bone, cartilage and tendons, but particularly in connective tissue. Methods for extracting collagen from tissue are well known.

The aim of the present invention is to produce from natural collagen (prepared using known methods) a biologically active support consisting of collagen which can release, both in aqueous solution and in contact with the skin, from 0.5% to 50% of its weight in natural soluble animal collagen. Such a support must have a denaturation temperature no lower than 37° C. Although this support is a new product, it has known cosmetological activity (as described below).

However, it does have novel aspects. It may be used for incorporation of cosmetically or pharmacologically active substances, with the advantage that the support may be stored with none of the denaturation or degradation problems encountered with similar known products.

The support matrix according to this invention, may be prepared in various forms (such as spheres, cylinders or sheets) which may due to its soft spongy or felt-like structure be cut into the size and thickness desired.

The presence of natural insoluble collagen, while not an active substance in itself, endows the support matrix with considerable firmness, facilitating handling during production and packaging as well as easy application and removal.

The product may be used for cosmetic or pharmacologic purposes by bringing suitable forms, eg: sheets, into contact with the desired area of the skin surface. The natural moisture of the skin then dissolves the soluble collagen and any other active substances, which can then exert their effects upon the skin.

While collagen support materials with a spongy or felt-like structure are already known, their denaturation temperature is too low, or they cannot release natural soluble collagen (either in aqueous solution or on contact with the skin) since they are produced using highly reticulated chemical binding substances. When treated with these chemicals, natural collagen is irreversibly reticulated so as to bind any natural soluble collagen almost completely. Furthermore, the presence of these binding or preservative substances is a contra-indication to their main cosmetic application of treatment of dry skin. They may, in fact, be quite damaging to the skin itself.

Attempts were made to solve this problem by using natural soluble collagen in oil/water emulsions, in aqueous or glycol solutions, or in other chemico-physical forms to be used either directly on the skin or by application on a reticulated collagen support at the moment of use. However these products suffer from the severe disadvantage that collagen in solutions or emulsions has an extremely brief and often indeterminant life cycle due to its rapid denaturation. Therefore, the use of preservatives is required and efficacy is even then not reliable.

The following publications described the techniques discussed above:
K. H. Stenzel, T. Miyata, A. L. Rubin,
Collagen as biomaterial
Ann. Rev. Biophys. Bioeng., 3, 231-253 (1974);
U.S.A. patent 3,823,212;
German patent 2625289;
A. Berg, H. Lindner,
Schutz-Proteine in der Kosmetik
Parf. & Kosm., 60, 74-78 (1979);
U.S.A patent 4,193,813;
A. Berg, H. Dieringer,
Collagen mask - Cosmetic Application & Analytical Control;
Atti Congresso Naz. SICC. Milano 24-25 Nov. 1983
M. Chvapil, Z. Eckmayer,
Role of proteins in cosmetics,
Int. Journal of Cos. Science, 7, 41-49 (1985)

The support matrix material, according to this invention, differs from previous collagen based materials in two ways:

First, it is charged with natural, soluble and stable collagen when it is packaged and it releases said collagen in high quantities on contact with the skin.

Second, since no preservatives or reticulating agents are used in the support material in this invention, there are none of the negative reactions which may ensue with similar previous products, such as irritation or rejection caused by trace residues of these preservatives or curing agents.

The advantages of this collagen support matrix material, according to the invention, are due above all to the fact that once the natural and soluble collagen is combined with the natural insoluble collagen (treated with no chemical reticulating agents) a final product is obtained which, when moistened or placed in contact with the skin, releases natural soluble collagen while the insoluble collagen fibers form a uniform, moist, semi-occlusive layer.

Thereby, action of the natural soluble collagen on the skin is facilitated, and the moisturizing process is enhanced, as has been shown in many publications on previous techniques. (Riso, R. R.: Protein derivatives - Moisture and 'That look of youth'. Aerosol Age, **14** 30 (1969); Riso, R. R.: Protein derivatives in cosmetics, Cosmet. Perfum. **89** 45-8 (1974); Nagelschmidt, M. and Struck, H.: Kollagen als Cosmeticum? Arch. Derm. Forsch. **250** 237-43 (1979); Richter, K.: Collagen (Losliches collagen contra unlosliches collagen). Information No. 328 in Richter, K. GmbH (Ed.) Chemisches Laboratorium (1971) (Adolph Furst & Sohn, Berlin); Tronnier, H.: Zur dermatologischen Wirksamkeit einer Kollagensalbe: Klinische-experimentelle Kurzmitteilung. Artzl. Kosmetologie **6** 93-5 (1976); Huc, A. et al,: Étude de la penetration in vivo dans la peau de rat du collagene acido-soluble en solution acide ou en melange dans une creme cosmetique. Inter. J. Cosmet. Sci. **3** 159-83 (1981); Todd, R. D.: Soluble collagen: new protein for cosmetics. Drug Cosmet. Indust. **117** 50-2, 56, 134-38 (1975); Groher, B.: Losliches Kollagen in kosmetischen Preparaten. Seifen Ole Fette Wachse **102** 499-500 (1976); Johnsen, V. L.: Proteins in cosmetics and toiletries. Drug Cosmet. Indust. **126** 36-9, 136 (1980).)

Another advantage is that in the dry state the support material may be stored with no addition of presevatives; in fact it may be sterilized with gamma radiation if suitably packaged. This sterilization is impossible with natural soluble collagen incorporated in emulsions or aqueous solutions. In fact, according to the invention, the natural soluble collagen in the support matrix remains stable for long periods, eve at high ambient room temperatures. This stability cannot be assured for products in aqueous solutions.

There are no other cosmetic products based on soluble collagen (such as emulsions, or aqueous or glycol solutions) which offer the guaranteed availability of such a high concentration of natural soluble collagen, with its inherent chemico-physical behaviour which allows complexation with other components and also stability.

Furthermore, the possibility of incorporating in the collagen matrix other cosmetically or pharmacologically active substances gives rise to the possibility of a product for local application to the skin which in addition to its moisturizing effects may also show therapeutic activity.

An object of the present invention is a process for the preparation of said support, consisting of the following operations:

- dissolving in distilled and/or sterilized water at room temperature a quantity of enriched natural soluble collagen prepared using known methods from biological tissue of young mammals, so as to obtain a solution with a concentration of from 5.0% to 40% by weight of collagen which does not denature at temperatures below 37 ° C.
- addition of a quantity of natural uncrosslinked insoluble collagen to the solution in a proportion ranging from 0.5:1 to 1:0.5 with respect to the soluble collagen, stirring to obtain a homogeneous mixture.
- rapid cooling of said mixture with stirring down to a temperature between 0 ° C and -5 ° C, so as to obtain a pourable mixture to be placed in forms for lyophilation.
- Lyophilation in said forms.

The invention is described below in greater detail in one preferred embodiment.

The support matrix, according to the invention, is prepared from a starting material consisting of concentrated natural and soluble animal collagen, obtained in successive separation and purification operations from biological tissue from young mammals, by means of acid extraction. This extraction process is already known. (Chvapil, M.: Process for the production of collagen fiber fabrics in the form of felt-like membranes or sponge-like layers - U.S. Patent 3. 823,212.9 (July 1974); Ries, P.E., Reinach: Verfahren zur Herstellung eines sterilen Kollagenproduktes mit filz- bzw. vliesartiger Faserstruktur. BRD Patent 2625289 (June 1976))

It should be noted that the denaturation temperature of collagen depends on the starting material used for extraction of the collagen itself. The starting material should therefore be selected on the basis of known criteria so that the collagen has a denaturation temperature higher than 37 ° C when it is prepared, preferably between 39 ° C and 45 ° C. Collagen denaturation, however, does not depend on temperature alone but on other factors as well, such as the presence of chemical substances.

Therefore, according to the present invention, the stable storage capacity of the soluble collagen does not depend directly on the natural denaturation temperature established by the choice of starting material, as mentioned above, but on the characteristics of the production process and material so obtained, unlike similar known products.

The preferred starting material for the soluble collagen is fresh tissue from young mammals, preferably in enriched solutions.

According to the invention, said mammalian source is dissolved in cold distilled and/or sterilized water, so as to achieve a solution with a concentration of active substance between 5.0% and 40% by weight with respect to the total solution. The solution is checked analytically to ensure that its denaturation temperature is not below 37°C.

The solution is preferably filtered to eliminate any impurities and the filtered solution is then treated with a homogenous suspension of insoluble animal collagen fibres, prepared using known methods. (Stenzel K.H., Miyata T., Rubin A. L.: Collagen as Biomaterial. Ann. Rev. Biophys. Bioeng. **3** 231-253 (1974); Chvapil, M.: Process for the production of collagen fiber fabrics in the form of felt-like membranes or sponge-like layers - U.S. Patent 3. 823,212.9 (July 1974); Ries, P.E., Reinach: Verfahren zur Herstellung eines sterilen Kollagenproduktes mit filz- bzw. vliesartiger Faserstruktur. BRD Patent 2625289 (June 1976))

The mixture is then stirred to ensure homogeneity and rapidly cooled, using refrigeration machines known in the cosmetic or pharmaceutical field for similar purpose.

The temperature is lowered until ice crystals form (between 0°C and -5°C) and the mixture is stirred so that the ice crystals are as finely divided as possible throughout the mixture. The mass must be sufficiently fluid to be poured into forms which serve to give the final shape to the support matrix material. The form selected is a function of the particular application for which the support matrix is to be used, as is evident to an expert in the field. The forms are then inserted in a lyophilation apparatus, in which the material is brought to its final appearance.

In this way the finished biologically active support matrix has an appearance that ranges from spongy to felt-like, and has the ability to release (in the presence of moisture) a quantity of from 0.5 to 50% of its weight as natural soluble collagen.

The support matrix, according to the invention, may be used as such for treatment of dry skin, since soluble collagen is known to produce a moisturizing effect, while the matrix containing the insoluble collagen acts as carrier and also as an occlusive wall to prevent dispersion of the active substance.

One important aspect of the present invention is that it is possible, during the soluble collagen solution phase or during the addition of insoluble collagen, to introduce cosmetically or pharmacologically active substances which are then readily released together with the soluble part of the collagen.

These active substances may be antibiotics, chemotherapeutics, antiseptics, cortisones, or any other active principle used pharmaceutically, biologically or cosmetically. Thus, the possibilities of utilization of the biologically active support matrix, according to the invention, are greatly enhanced. In the way, local treatment of both healthy skin (cosmetic treatment) and of unhealthy skin is possible, as is clear to an expert in this field.

Furthermore, the optimal storage properties of the product allow any active substances to be used in relatively low concentrations, resulting in savings in terms of costs.

It is clear that modifications or variations may be made to the support matrix, according to the invention, without going beyond the bounds of the invention itself.

## Claims

1. A process for the preparation of a support sponge matrix containing releasable soluble collagen, characterized by the steps of:

   (a) dissolving, in distilled and/or sterilized water at room temperature, a quantity of enriched natural soluble collagen prepared using known methods from biological tissues of young mammals, so as to obtain a solution having a concentration from 5 % to 40 % by weight of collagen which does not denature at temperatures below about 37° C;

   (b) adding a quantity of natural uncrosslinked insoluble collagen to the solution in a proportion by weight ranging from 0.5:1 to 1:0.5 with respect to the soluble collagen of step a;

   (c) stirring to obtain a homogeneous mixture;

   (d) during the continued stirring of said mixture, rapidly cooling the mixture to a temperature from 0° C to -5° C, so as to obtain a pourable mixture to be placed in forms for lyophilization;

   (e) lyophilizing the mixture in said forms; and

   (f) optionally, adding cosmetically or pharmacologically active substances to the mixture during any of the steps (a) through (c), inclusive.

2. A process according to claim 1, wherein the soluble collagen solution is filtered to remove any impurities before the addition of the natural uncrosslinked insoluble collagen.

3. A support sponge matrix containing releasable soluble collagen prepared by the process defined in claim 1.

4. A support sponge matrix containing releasable soluble collagen, comprising a natural uncrosslinked insoluble collagen matrix, and a soluble natural releasable collagen within the matrix, wherein the weight ratio of insoluble collagen to soluble collagen is 0.5:1 to 1:0.5, the soluble collagen is soluble in water, and having a denaturation temperature from 37° C to 45° C.

5. The support sponge matrix containing releasable soluble collagen, according to claim 4, further comprising cosmetically or pharmacologically active substances.

**Patentansprüche**

1. Ein Verfahren zur Herstellung einer schwammartigen, lösliches, freisetzbares Kollagen enthaltenden Trägermatrix, dadurch gekennzeichnet, dass man
   (a) eine Menge von angereichertem natürlichen, löslichen, auf bekannte Weise aus biologischen Geweben von jungen Säugetieren hergestellen Kollagen bei Zimmertemperatur in distilliertem oder sterilisierten Wasser löst, um eine Lösung mit 5 bis zu 40% Gewichtsprozent Kollagen zu erhalten, das bei Temperaturen unter circa 37°C nicht vergällt;
   (b) der Lösung eine Menge von unvernetztem und unlöslichen, natürlichen Kollagen in einem Gewichtsverhältniss von 0.5:1 bis zu 1:0.5 in Bezug auf das lösliche Kollagen in Schritt (a) zufügt;
   (c) durchrührt um eine gleichmässige Mischung zu erhalten;
   (d) bei dauerndem Rühren die Mischung bis auf eine Temperatur von 0°C bis -5°C abkühlt, um eine vergiessbare Mischung zu erhalten, die in Liophilisationsgefässe gegossen werden kann;
   (e) die Mischung in den Gefässen liophilisiert, und
   (f) wahlweise, der Mischung während der Schritte (a) bis (c), einschliesslich, kosmetisch oder pharmazeutisch aktive Stoffe zusetzt.

2. Ein Verfahren nach Anspruch 1, in dem die Lösung aus löslichem Kollagen filtriert wird, um Unreinheiten zu beseitigen bevor das natürliche, unvernetzte und unlösliche Kollagen beifügt wird.

3. Eine mit einem in Anspruch 1 beschriebenen Verfahren hergestellte, lösliches, freisetzbares Kollagen enthaltende schwammartige Trägermatrix.

4. Eine lösliches, freisetzbares Kollagen enthaltende schwammartige Trägermatrix, bestehend aus einer Matrix aus unlöslichem, unvernetzten Kollagen, und aus natürlichem, löslichen, freisetzbaren Kollagen innerhalb der Matrix, worin das Gewichtsverhältniss von unlöslichem Kollagen zu löslichem Kollagen von 0.5:1 bis zu 1:0.5 ist, das lösliche Kollagen wasserlöslich ist und eine Vergällungstemperatur von 37°C bis 45°C hat.

5. Die lösliches, freisetzbares Kollagen enthaltende schwammartige Trägermatrix nach Anspruch 4, die weiterhin kosmetisch oder pharmazeutisch aktive Stoffe enthält.

**Revendications**

1. Procédé pour la préparation d'une matrice spongieuse de support contenant du collagène soluble libérable, caractérisé par les opérations suivantes:
   (a) on dissoud dans de l'eau distillée et/ou stérilisée à la température ambiante, une quantité de collagène naturel soluble enrichi préparé selon des méthodes connues à partir de tissus biologiques de jeunes mammifères pour obtenir une solution ayant une concentration de 5 à 40 pourcent en poids de collagène qui ne se dénature pas à des températures inférieures à environ 37°C;
   (b) on ajoute une quantité de collagène insoluble naturel non réticulé à la solution, dans une proportion en poids de 0,5:1 à 1:0,5 par rapport au collagène soluble de l'opération (a);
   (c) on mélange pour obtenir un mélange homogène;
   (d) pendant le mélangement continu dudit mélange, on refroidit rapidement le mélange à une température de 0 à -5°C pour obtenir un mélange coulable qui peut être mis dans les moules pour la lyophilisation;
   (e) on lyophilise le mélange dans ces moules; et
   (f) éventuellement, on ajoute des substances cosmétiquement ou pharmaceutiquement actives au mélange pendant une quelconque des opérations de (a) à (c) y inclus.

2. Procédé selon la revendication 1, où la solution de collagène soluble est filtrée pour éliminer toute impurité avant l'adjonction du collagène naturel insoluble non réticulé.

3. Matrice spongieuse de support contenant du collagène soluble libérable préparée par le procédé défini dans la revendication 1.

4. Matrice spongieuse contenant du collagène soluble libérable comprenant une matrice de collagène naturel insoluble non réticulée et un collagène naturel soluble libérable dans la matrice, où le rapport en poids du collagène insoluble par rapport au collagène soluble est 0,5:1 à 1:0,5, le collagène soluble est soluble dans l'eau et il a une température de dénaturation de 37°C à 45°C.

5. Matrice spongieuse contenant du collagène soluble libérable selon la revendication 4, contenant en outre des substances cosmétiquement et pharmacologiquement actives.